# EUROPEAN PATENT SPECIFICATION

(11) **EP 2 784 507 B1**
(45) Date of publication and mention of the grant of the patent: **02.12.2020**
(21) Application number: 14162324.9
(22) Date of filing: 28.03.2014
(51) Int. Cl.: G01N 33/543, G01N 33/58

(54) **Test substance assay method, test substance assay kit, and test substance assay reagent**
Testsubstanztestverfahren, Testsubstanztestkit und Testsubstanztestreagenz
Procédé de dosage d'une substance d'essai, kit de dosage d'une substance d'essai et réactif de dosage d'une substance d'essai

(30) Priority: 29.03.2013 JP 2013071030; 04.09.2013 JP 2013182889; 10.02.2014 JP 2014023109
(43) Date of publication of application: 01.10.2014
(73) Proprietor: FUJIFILM Corporation, Minato-ku Tokyo 106-8620 (JP)
(72) Inventor: Kasagi, Noriyuki, Kanagawa, 258-8577 (JP); Chiku, Hiroyuki, Kanagawa, 258-8577 (JP); Ujihara, Dai, Kanagawa, 258-8577 (JP); Yoshitani, Toshihide, Kanagawa, 258-8577 (JP)
(74) Representative: Hoffmann Eitle

(56) References cited:
- WO-A1-2012/099897
- US-A- 5 843 651
- US-A1- 2013 078 738
- O'Farrell B.: "Lateral flow immunoassay systems: Evolution from the current state of the art to the next generation of highly sensitive, quantitative rapid assays" In: Wild D.G.: "The Immunoassay Handbook, 4th Edition", 31 January 2013 (2013-01-31), Elsevier, XP55132095, ISBN: 978-0-08-097037-0 pages 89-107, DOI: 10.1016/B978-0-08-097037-0.00007-5, * abstract * * page 92, column 1, last paragraph * * page 93, column 1, paragraph 1-2 * * page 98, column 2 * * page 100, column 1, paragraph 2 - page 103, column 1, paragraph 1 *
- SIIMAN O. ET AL.: "Preparation, microscopy, and flow cytometry with excitation into surface plasmon resonance bands of gold or silver nanoparticles on aminodextran-coated polystyrene beads", J. PHYS. CHEM. B, vol. 104, no. 42, 2000, pages 9795-9810, XP008071161,

## Description

### BACKGROUND OF THE INVENTION

### 1. Field of the Invention

The present invention relates to a test substance assay method based on an antigen-antibody reaction using insoluble carriers, a kit for performing a test substance assay and a test substance assay reagent used for implementing the method.

### 2. Description of the Related Art

Conventionally, immunoassay, which is a method of assaying a trace of test substance as a specific component in a specimen such as a biological sample including blood and the like, is widely used in the field of clinical examination. In the immunoassay, for example, a specific reaction represented by an antigen-antibody reaction is used, and such a reaction is used as extremely useful means that makes it possible for a test substance of interest to be measured specifically. However, there are test samples that test positive by reacting not only with test samples which contain a test substance and will test positive but also with test samples which do not contain a test substance and will test negative, and results in false positive. This has been conventionally regarded as being a problem. The cause of resulting in false positive as above is unclear; however, it is assumed that a certain factor contained in blood may be one of the factors causing a non-specific reaction.

As a technique for suppressing the non-specific reaction, JP1985-256057A (JP-S60-256057A) discloses the immunoassay, particularly, the immunoassay utilizing agglutination, in which ultrafine particles having a particle size of equal to or smaller than 0.2 µm are used to hinder a non-specific immune reaction of sensitized particles having a particle size of 0.3 µm to 2.0 µm. JP2000-221196A discloses a method of detecting a test substance by an immunoagglutination reaction using sensitized particles having a particle size of equal to or greater than 0.4 µm, in which insoluble carrier particles having a particle size of 0.01 µm to 0.5 µm are used as particles for blocking. Moreover, JP3623657B discloses, for the purpose of suppressing the non-specific reaction, a method of adding particles which are smaller than particles specifically reacting with a substance to be measured and to which an antigen or an antibody that does not immunologically react with the aforementioned substance has been fixed. Furthermore, JP2007-127438A discloses a non-specific reaction inhibitor used for the immunoassay using immunoassay particles that are obtained by causing an antibody or an antigen, which immunologically reacts with a substance to be measured, to be supported on carriers having an average particle size of 0.05 µm to 0.5 µm. According to JP2007-127438A, the non-specific reaction inhibitor is formed of insoluble carriers that are caused to support the antigen or an antibody, which does not immunologically reacts with the substance to be measured, in the presence of an organic solvent, and the average particle size of the insoluble carriers is smaller than the average particle size of the aforementioned carriers. JP2010-19553A discloses a detection method for distinguishing specific binding reactions from non-specific binding reactions that are caused between various biological molecules, in which the influence of the non-specific reaction is diminished using particles having an outer diameter of 1 µm or less.

Meanwhile, a method which uses the effect of electric field enhancing effect produced by plasmon resonance to improve detection sensitivity of the immunoassay is known. In this method, in order to cause the plasmon resonance, a sensor chip obtained by disposing a metal layer in a predetermined area on a transparent support is prepared, and excitation light is caused to enter the interface between the support and the metal film from the support surface opposite to the support surface in which the metal layer is formed, at a predetermined angle that is equal to or greater than the angle of total reflection. By the irradiation of the excitation light, surface plasmon is caused in the metal layer. Since the surface plasmon caused in this manner enhances electric field, fluorescence is enhanced, whereby a signal/noise (S/N) ratio increases. In a fluorimetric detection method implemented by surface plasmon excitation (hereinafter, referred to as a "SPF method"), signal intensity becomes about 10 times higher than in a fluorimetric detection method implemented by epi-illumination excitation (hereinafter, referred to as an "epi-illumination fluorimetry"), and accordingly, a test substance can be measured with high sensitivity.

For example, in the optical signal detection method which is disclosed in JP2010-190880A and determines the amount of a test substance, a sensor chip having a sensor portion including a metal layer in which a dielectric plate is disposed in a predetermined area of one surface thereof is prepared, and a sample is brought into contact with the sensor portion of the sensor chip. By the contact, a binding substance labeled with a photoresponsive labeling substance that is in an amount corresponding to the amount of the test substance contained in the sample binds to the sensor portion. Thereafter, a predetermined area is irradiated with excitation light, and light from the photoresponsive labeling substance that is generated in an electric field enhancement site formed on the metal layer is detected, whereby the amount of the test substance is determined. In this method, as the photoresponsive labeling substance, it is possible to use plural photoresponsive substances contained in a light transmissive material that transmits light generated from the photoresponsive substance, such that metal-induced quenching caused when the photoresponsive substance approaches the metal layer is prevented.

In addition, JP2010-112748A discloses a detection method in which a sensor chip including a sensor portion having a laminate structure consisting of a dielectric plate and a metal layer which is on one surface of the dielectric plate and adjacent to the dielectric plate is used; a sample is brought into contact with the sensor portion such that a fluorescence-labeled binding substance, which is in an amount corresponding to the amount of a test substance to be detected contained in the sample and consists of a fluorescent label and a binding substance labeled with the fluorescent label, binds to the top of the sensor portion; the sensor portion is irradiated with excitation light to generate an enhanced optical electric field on top of the sensor portion; and the fluorescent label is excited with the enhanced optical electric field so as to detect the amount of the substance to be detected based on the amount of light generated by the excitation. The detection method uses, as the fluorescent label, a fluorescent substance consisting of plural first fluorescent dye molecules and first particles which are formed of light transmissive material transmitting fluorescence generated from the plural first fluorescent dye molecules and contain the plural first fluorescent dye molecules. Furthermore, the detection method also uses, as a blocking agent for preventing adsorption of the fluorescence-labeled binding substance onto the sensor portion that is caused by non-specific adsorptivity of the fluorescence-labeled binding substance with respect to the sensor portion, a blocking substance which does not contain the first fluorescent dye molecules, does not non-specifically bind to the binding substance, and has non-specific adsorptivity equivalent to the non-specific adsorptivity of the fluorescence-labeled binding substance.

US-A-2013/0078738 discloses a method for measuring a test substance which comprises the steps:
(1) reacting fluorescent particles and the test substance by bringing the test substance and the fluorescent particles into contact with a test area and a control area on a substrate,
(2) measuring fluorescence of the fluorescent particles of the test area and the control area, and
(3) correcting a fluorescence signal value in the test area using a fluorescence signal value in the control area, wherein the fluorescent particles are binding substance-labeling fluorescent particles in which (i) one or more of a first binding substance which can bind to the test substance, and (ii) a third binding substance which can bind to a second binding substance and does not bind to the test substance are bound, and wherein the second binding substance which can bind to the first binding substance is immobilized on the control area.

Example 1 of this reference illustrates such a method using fluorescent latex particles having an average diameter of 200 nm conjugated to an anti-cortisol antibody.

### SUMMARY OF THE INVENTION

The immunoassay needs to have high sensitivity and high reproducibility. It also needs to enable assay to be performed with high accuracy and needs to exhibit excellent storability. Moreover, for making diagnosis in medical practice, the immunoassay is required to be convenient to such a degree that enables each medical office to perform assay, and required to have performance that enables the assay to be completed in a short time such that the assay result can be checked on the spot. In the techniques disclosed in JP1985-256057A (JP-S60-256057A), JP2000-221196A, JP3623657B, JP2007-127438A, and JP2010-19553A, as the particle suppressing non-specific adsorption, particles having a small particle size and a large specific surface area can be used, and accordingly, the substance causing non-specific adsorption can be trapped with high efficiency. However, these techniques relate to the method of detecting a test substance by means of immunoagglutination and detect the change in turbidity resulting from agglutination of particles that is caused when a sample contains the test substance. Therefore, in order to increase sensitivity, these techniques must use particles detecting a large test substance. Consequently, depending on the storage condition or the timing of assay, the reproducibility of assay, storability, and the like have problems due to precipitation, agglutination, and the like of the particles.

An object of the present invention is to provide a test substance assay method which has high sensitivity, can avoid false positives by suppressing non-specific reactions of a test substance, and has excellent reproducibility of assay. Another object of the present invention is to provide a kit for performing a test substance assay and a test substance assay reagent which are used for implementing the test substance assay method.

As a result of conducting thorough examination to achieve the above objects, the present inventors found out by applying a mixed solution, which is obtained by mixing (a) first dry particles that are modified with first binding substances having binding properties specific to a test substance, have an average particle size of 100 nm to 200 nm, and have labels and (b) second dry particles that are modified with second binding substances not having binding properties specific to the test substance, have an average particle size of 100 nm to 200 nm, and do not have labels with a test sample solution containing the test substance, onto a substrate, causing the test substance to be trapped in a reaction site on a substrate that has third binding substances having binding properties specific to the test substance or has substances having binding properties specific to the test substance, and detecting the test substance trapped in the reaction site, it is possible to measure the test substance with high sensitivity and excellent reproducibility of the assay while avoiding false positives by suppressing non-specific reactions of the test substance. The present invention has been made based on the above findings.

That is, according to the present invention, there is provided a test substance assay method including:
(i) forming a mixed solution by mixing (a) first dry particles that are fluorescent latex particles with an average particle size of 100 nm to 200 nm and modified with first binding substances which are antibodies, antigens or complexes of these having binding properties specific to a test substance, and (b) second dry particles that are non-fluorescent latex particles with an average particle size of 100 nm to 200 nm and modified with second binding substances which are antibodies not having binding properties specific to the test substance, with (c) a test sample solution containing the test substance, the mass ratio of the second dry particles to the first dry particles being 2:1 to 6:1;
(ii) applying the mixed solution obtained in the step (i) onto a substrate having a reaction site, the reaction site having third binding substances which are antibodies, antigens or complexes of these with binding properties specific to the test substance; or has substances exhibiting binding properties with respect to the first binding substances;
(iii) causing the test substance to be trapped in the reaction site on the substrate; and
(iv) detecting the test substance trapped in the reaction site.

Moreover, according to the present invention, there is provided a kit for performing a test substance assay comprising:
first dry particles that are fluorescent latex particles with an average particle size of 100 nm to 200 nm and modified with first binding substances which are antibodies, antigens or complexes of these having binding properties specific to a test substance;
second dry particles that are non-fluorescent latex particles with an average particle size of 100 nm to 200 nm and modified with second binding substances which are antibodies not having binding properties specific to the test substance, the mass ratio of the second dry particles to the first dry particles being 2:1 to 6:1;
a container containing the first dry particles and the second dry particles;
a channel through which the first dry particles and the second dry particles are capable of flowing; and
a substrate that has third binding substances which are antibodies, antigens or complexes of these having binding properties specific to the test substance; or has substances exhibiting binding properties with respect to the first binding substances.

Furthermore, according to the present invention, there is provided the test substance assay method that is implemented using the kit for test substance assay according to the present invention.

In addition, according to the present invention, there is provided a test substance assay reagent comprising:
(a) first dry particles that are fluorescent latex particles with an average particle size of 100 nm to 200 nm and modified with first binding substances which are antibodies, antigens or complexes of these having binding properties specific to a test substance; and
(b) second dry particles that are non-fluorescent latex particles with an average particle size of 100 nm to 200 nm and modified with second binding substances which are antibodies not having binding properties specific to the test substance, the mass ratio of the second dry particles to the first dry particles being 2:1 to 6:1.

The first binding substances having binding properties specific to the test substance are preferably antibodies.

The first binding substances having binding properties specific to the test substance are preferably antibodies derived from a mouse.

It is preferable that in the step (iv), the test substance trapped in the reaction site be detected by surface plasmon fluorimetry.

According to the test substance assay method of the present invention, the test substance can be measured with high sensitivity, false positives can be avoided by suppressing non-specific reactions of the test substance, and reproducibility of the assay becomes excellent. Likewise, in a test substance assay method using the test substance assay kit and the test substance assay reagent of the present invention, the test substance can be measured with high sensitivity, false positives can be avoided by suppressing non-specific reactions of the test substance, and reproducibility of the assay becomes excellent.

### DESCRIPTION OF THE PREFERRED EMBODIMENTS

Hereinafter, the present invention will be described in more detail.

The test substance assay method of the present invention includes:
(i) a step of obtaining a mixed solution by mixing (a) first dry particles that are modified with first binding substances exhibiting binding properties specific to a test substance, have an average particle size of 100 nm to 200 nm, and have labels, and (b) second dry particles that are modified with second binding substances not exhibiting binding properties specific to the test substance, have an average particle size of 100 nm to 200 nm, and do not have labels with (c) a test sample solution containing the test substance;
(ii) a step of applying the mixed solution obtained in the step (i) onto a substrate;
(iii) a step of causing the test substance to be trapped in a reaction site on the substrate that has third binding substances having binding properties specific to the test substance or has substances exhibiting binding properties with respect to the first binding substances; and
(iv) a step of detecting the test substance trapped in the reaction site.

The present invention particularly has characteristic points including a point (the first characteristic) that the invention uses the second particles that are modified with the second binding substances not having binding properties specific to the test substance, have an average particle size of 100 nm to 200 nm, and do not have labels; and a point (the second characteristic) that both the first particles, which are modified with the first binding substance having binding properties specific to the test substance, have an average particle size of 100 nm to 200 nm, and have labels, and the second particles, which are modified with the second binding substances not having binding properties specific to the test substance, have an average particle size of 100 nm to 200 nm, and do not have labels, are dry particles, and these two kinds of particles are used by being mixed with the test sample solution containing the test substance. The aforementioned first characteristic makes it possible to suppress non-specific reactions of the test substance. Accordingly, false positives can be avoided, and the test substance can be measured with high sensitivity. Moreover, by the aforementioned second characteristic, reproducibility of the assay becomes excellent. If the particles are stored in a solution, the particle size may increase due to agglutination, or reproducibility thereof may deteriorate. According to the present invention, the above problems are solved by storing the particles in a dry state.

### (First dry particles and second dry particles)

The first particles are fluorescent latex particles and the second particles used in the present invention are stored in a dry state and used at the time of assay by being mixed with a test sample solution containing a test substance. When the first particles and the second particles are stored in the state of a solution, agglutination or fusion is caused among the particles, and accordingly, the particle size increases, and accuracy of assay changes in some cases. Therefore, in the present invention, in order to avoid such problems, the first particles and the second particles are stored in a dry state. In this case, in order to maintain reproducibility of the assay at an excellent level, not only the second particles but also the first particles are required to have an average particle size of 100 nm to 200 nm. That is, in the present invention, both the first particles having labels and the second particles have an average particle size of 100 nm to 200 nm. If the average particle size of the particles exceeds 200 nm, when being mixed with the test sample solution containing the test substance, the particles may poorly dissolve in the solution in some cases, whereby reproducibility of the assay or reproducibility of the ability to prevent false positives may deteriorate. Moreover, if the average particle size of the particles is less than 100 nm, signal sensitivity becomes insufficient, and the effect of the present invention that maintains reproducibility of the assay at an excellent level cannot be obtained. The average particle size of the first particles and the second particles of the present invention is more preferably from 100 nm to 190 nm and even more preferably from 130 nm to 180 nm.

The mass ratio of the second dry particles to the first dry particles is 2:1 to 6:1.

### (Method of assaying average particle size)

The average particle size of the first dry particles and the second dry particles used in the present invention can be measured by commercially available particle size distribution analyzers and the like. As methods of assaying particle size distribution, the methods using an optical microscope, a confocal laser microscope, an electron microscope, and an atomic force microscope, a static light scattering method, a laser diffraction method, a dynamic light scattering method, a centrifugal precipitation method, an electric pulse assay method, chromatography, an ultrasonic attenuation method, and the like are known, and apparatuses corresponding to principles of the respective methods are commercially available.

From the viewpoints of the range of particle size and ease of assay, a dynamic light scattering method can be preferably used in the present invention. Examples of commercially available assay apparatuses using the dynamic light scattering method include Nanotrack UPA (NIKKISO CO., LTD.), a dynamic light scattering-type particle size distribution analyzer LB-550 (HORIBA, Ltd.), a concentrated system-particle size analyzer FPAR-1000 (OTSUKA ELECTRONICS CO., LTD.), and the like. In the present invention, a value of median diameter (d = 50) measured at 25°C can be taken as the average diameter.

### (Latex particles)

The material of the first dry particles and the second dry particles used in the present invention is not particularly limited as long as the effects of the present invention can be obtained. Latex particles are used as the material. Specific examples of the material of latex include polystyrene, styrene-acrylic acid copolymers, styrene-methacrylic acid copolymers, styrene-glycidyl (meth)acrylate copolymers, styrene-styrene sulfonate copolymers, methacrylic acid copolymers, acrylic acid copolymers, acrylonitrile-butadiene-styrene copolymers, vinyl chloride-acrylic acid ester copolymer, polyvinyl acetate acrylate, and the like. As the latex, copolymers that contain at least styrene as a monomer are preferable, and copolymers of styrene and acrylic acid or methacrylic acid are particularly preferable.

The method of preparing the latex is not particularly limited, and any polymerization of preparing the latex methods can be used. However, when antibody labeling is performed in the presence of a surfactant, it is difficult to conduct antibody immobilization. Accordingly, it is preferable to perform soap-free polymerization for preparing the latex.

In a particularly preferable embodiment, the latex particles used in the present invention contain styrene and acrylic acid or methacrylic acid and can be produced by performing polymerization by means of adding dropwise a polymerization initiator to an aqueous suspension having a styrene concentration of 1.4 M or lower. The use of an aqueous suspension having a styrene concentration of 1.4 M or higher is not preferable since the latex particles generated in the polymerization system bind to one another and thus the average particle size of the generated latex particles increases. Moreover, it is preferable to increase the temperature of the aqueous suspension to 75°C to 100°C before the polymerization initiator is added. Increasing the temperature in this manner is preferable since the initiator can be decomposed as soon as being added to the suspension, radicals can be generated in an instant in the polymerization system, and consumption of monomers can be started, whereby the effect of making the latex particle size uniform is obtained.

The polymerization initiator is not particularly limited as long as it can initiate polymerization, and any known polymerization initiators can be used. For example, potassium persulfate (KPS), 2,2'-azobisisobutyronitrile, 2,2'-azobis(2,4-dimethyl)valeronitrile, 2,2'-azobis(4-methoxy-2,4-dimethylvaleronitrile), benzoyl peroxide, 2,4-dichloroperoxide, isopropyl peroxycarbonate, cumene hydroperoxide, lauroyl peroxide, and the like can be used for polymerization. It is particularly preferable to perform the polymerization using potassium persulfate. The amount of the polymerization initiator used is preferably about 0.1% by mass to 5% by mass of the monomer composition.

The polymerization can also be performed in the presence of a crosslinking agent. As the crosslinking agent, for example, divinyl benzene or 1,4-butadiene can be used, but the crosslinking agent is not limited to these.

As methods of preparing the latex particles of which the average particle size is within the range specified by the present invention, the concentration of the monomer or initiator or the polymerization temperature can be adjusted.

### (First dry particles having labels)

The first dry particles of the present invention are latex particles having detectable fluorescent labels. If the latex obtained by polymerization is fluorescent, the latex can be directly used as fluorescent latex particles. If the latex obtained by polymerization is not fluorescent, by adding a fluorescent substance (a fluorescent dye or the like) to the latex, fluorescent latex particles can be prepared. That is, the fluorescent latex particles can be produced by adding a fluorescent dye to a latex particle solution containing water and a water-soluble organic solvent and stirring the resultant. The latex concentration in the latex particle solution is preferably 0.1% by mass to 10% by mass. It is preferable for the solution to contain an electrolyte. As the electrolyte, NaCl is preferable, and the electrolyte concentration in the solution is preferably 1 mM to 500 mM. In addition, as the water-soluble organic solvent contained in the latex particle solution, tetrahydrofuran (THF), dimethylformamide (DMF), dimethylacetamide (DMAc), or acetone is preferable. The proportion of water and the water-soluble organic solvent is preferably about 10% by mass to 80% by mass.

As described above, the "fluorescent latex particles" described in the present specification includes both the fluorescent latex particles that are obtained when the latex obtained by polymerization is fluorescent, and the fluorescent latex particles that are obtained by adding a fluorescent substance (a fluorescent dye or the like) to non-fluorescent latex which is obtained by polymerization.

### (Particles modified with binding substances)

The first dry particles of the present invention are modified with the first binding substances having binding properties specific to a test substance. Moreover, the second dry particles of the present invention are modified with the second binding substances not having binding properties specific to the test substance.

### (Test substance)

The type of the test substance which is to be detected by the assay method of the present invention is not particularly limited. Examples of the test substance include cortisol, insulin-like growth factor-1 (IGF-1), insulin-like growth factor-binding protein-3 (IGFBP-3), luteinizing hormone (LH), thyroid-stimulating hormone (TSH), antidiuretic hormone (ADH), growth hormone (GH), urinary growth hormone, adrenocorticotropic hormone (ACTH), prolactin, follicle-stimulating hormone (FSH), tyrosine-binding globulin (TBG), TSH-stimulating receptor antibodies (TSAb), tyrosine (T4), anti-thyroid peroxidase antibodies (anti-TPO antibodies), microsomal antibodies, anti-thyroglobulin antibodies, thyroglobulin, triiodothyronine (T3), fT4, fT3, 1,25-(OH)2 vitamin D, type I collagen crosslinked N-telopeptide (NTx), intact type I procollagen-N-propeptide (Intact PINP), osteocalcin, calcitonin, bone-specific alkaline phosphatase, (BAP), deoxypyridinoline, parathyroid hormone (PTH), parathyroid hormone-related protein (PTHrP), 5-hydroxyindoleacetic acid (5-HIAA), homovanillic acid (HVA), L-dopa, 3-methoxy-4-hydroxyphenyl ethylene glycol (MHPG), vanillylmandelic acid (VMA), catecholamine, serotonin, metanephrine, 11-deoxycortisol, 17-ketogenic steroid (17-KGS), 17-OH-pregnenolone, aldosterone, androsterone, androstenedione, 11-hydroxycorticosteroid (11-OHCS), corticosterone, cortisone, deoxycorticosterone (DOC), dehydroxyepiandrosterone sulfate (DHEA-S), pregnenolone, 5α dihydrotestosterone, human chorionic gonadotropin (HCG) β subunit, estradiol (E2), estriol (E3), estrogen, estrone (E1), human chorionic gonadotropin (HCG), testosterone, pregnanediol, pregnanetriol, progesterone, C peptide (CPR), vasoactive intestinal peptide (VIP), insulin, gastrin, glucagon, anti-glutamic acid decarboxylase antibodies (anti-GAD antibodies), anti-insulinoma-associated antigen-2 antibodies (anti-IA-2antibodies), anti-insulin antibodies, cardiac troponin T, ventricular myosin light chain I, heart-type fatty acid-binding protein (H-FABP), human atrial natriuretic peptide (HANP), brain natriuretic peptide (BNP), n-terminal prohormone of brain natriuretic peptide (NT-proBNP), myoglobin, and the like. A particularly preferable example of the test substance is TSH.

### (First binding substances)

The first dry particles of the present invention are modified with the first binding substances having binding properties specific to the test substance. The first binding substances are antibodies, antigens, and complexes of these. For example, when the binding substances are antibodies, as the antibodies exhibiting specificity for the test substance, for example, antiserum prepared from the serum of an animal immunized with the test substance, an immunoglobulin fraction purified from antiserum, monoclonal antibodies obtained by cell fusion using splenocytes of an animal immunized with the test substance or fragments thereof (for example, F(ab')2, Fab, Fab', or Fv), and the like can be used. These antibodies can be prepared by conventional methods. Moreover, these antibodies may be modified antibodies such as chimeric antibodies, and it is also possible to use both the commercially available antibodies and the antibodies prepared from animal serum or culture supernatant by known methods.

The antibodies can be used regardless of the type of animal, the subclass thereof, and the like. For example, the antibodies that can be used in the present invention include immunoreactive antibodies derived from living organisms such as a mouse, a rat, a hamster, a goat, a rabbit, a sheep, a cow, and a chicken. The antibodies specifically include mouse IgG, mouse IgM, rat IgG, rat IgM, hamster IgG, hamster IgM, rabbit IgG, rabbit IgM, goat IgG, goat IgM, sheep IgG, sheep IgM, cow IgG, cow IgM, chicken IgY, and the like, and both the polyclonal and monoclonal antibodies can be used. The antibody fragment is a molecule which has at least one antigen-binding site and derived from a complete antibody, and specific examples thereof include Fab, F(ab')2, and the like. These antibody fragments are obtained by enzymatic treatment or chemical treatment or using gene engineering technique.

The method of immobilizing the binding substances such as antigens or antibodies onto particles are disclosed in, for example, JP 2000-206115 A, the protocol attached to FluoSpheres (registered trade mark) polystyrene microspheres F8813 from Molecular Probes, or the like, and any of known methods that prepare reagents for an immunoagglutination reaction can be used. Moreover, as the principle of immobilizing antibodies as binding substances onto particles, any of principles of physical adsorption and chemical bonding established by covalent bonds can be adopted. As a blocking agent for covering the particle surface not covered with the antibody even after the antibodies are immobilized onto the particles, known substances, for example, bovine serum albumin (BSA), skim milk, casein, soybean-derived components, fish-derived components, polyethylene glycol, or commercially available blocking agents for immune reaction that contain the above substances or contain substances having the same property as that of the above substances can be used. These blocking agents can be optionally subjected to pretreatment such as partial denaturation by means of heat, an acid, an alkali, and the like.

### (Second binding substances)

The second dry particles of the present invention that do not have labels are modified with the second binding substances not having binding properties specific to the test substance. The second binding substances are binding substances (antibodies) and do not exhibit affinity for the first binding substances. Antiserum prepared from the serum of an animal immunized with the test substance, an immunoglobulin fraction purified from antiserum, monoclonal antibodies obtained by cell fusion using splenocytes of an animal immunized with the test substance or fragments thereof (for example, F(ab')2, Fab, Fab', or Fv) can be used. These antibodies can be prepared by conventional methods. Moreover, these antibodies may be modified antibodies such as chimeric antibodies, and it is also possible to use both the commercially available antibodies and the antibodies prepared from animal serum or culture supernatant by known methods.

The method of immobilizing the second binding substances such as antibodies onto particles are disclosed in, for example, JP 2000-206115 A, the protocol attached to FluoSpheres (registered trade mark) polystyrene microspheres F8813 from Molecular Probes, or the like, and any of known methods that prepare reagents for an immunoagglutination reaction can be used. Moreover, as the principle of immobilizing antibodies as binding substances onto particles, any of principles of physical adsorption and chemical bonding established by covalent bonds can be adopted. As a blocking agent for covering the particle surface not covered with the antibody even after the antibodies are immobilized onto the particles, known substances, for example, bovine serum albumin (BSA), skim milk, casein, soybean-derived components, fish-derived components, polyethylene glycol, or commercially available blocking agents for immune reaction that contain the above substances or contain substances having the same property as that of the above substances can be used. These blocking agents can be optionally subjected to pretreatment such as partial denaturation by means of heat, an acid, an alkali, and the like.

### (Third binding substances and substances exhibiting binding properties with respect to the first binding substances)

In the present invention, the third binding substances having binding properties specific to the test substance or substances having binding properties specific to the first biding substances are immobilized onto a substrate to form a reaction site. The third binding substances immobilized onto the reaction site are antigens, antibodies, or complexes of these. For example, when the binding substances are antibodies, as antibodies exhibiting specificity for the test substance, for example, antiserum prepared from the serum of an animal immunized with the test substance, an immunoglobulin fraction purified from antiserum, monoclonal antibodies obtained by cell fusion using splenocytes of an animal immunized with the test substance or fragments thereof (for example, F(ab')2, Fab, Fab', or Fv), and the like can be used. These antibodies can be prepared by conventional methods. Moreover, these antibodies may be modified antibodies such as chimeric antibodies, and it is also possible to use both the commercially available antibodies and the antibodies prepared from animal serum or culture supernatant by known methods. In addition, when the test substance is antigens, and both the first and third binding substances are antibodies, the first and third binding substances are antibodies against the same antigens, but each of the first and third binding substances recognizes different epitopes.

Examples of the substances exhibiting binding properties with respect to the first binding substances include the test substance or substances which have a site similar to the test substance and have an epitope that is recognized by the first binding substances similarly to the test substance.

Antibodies can be used regardless of the animal species, the subclass, and the like. For example, the antibodies that can be used in the present invention include immunoreactive antibodies derived from living organisms such as a mouse, a rat, a hamster, a goat, a rabbit, a sheep, a cow, and a chicken. The antibodies specifically include mouse IgG; mouse IgM, rat IgG; rat IgM, hamster IgG; hamster IgM, rabbit IgG; rabbit IgM, goat IgG; goat IgM, sheep IgG; sheep IgM, cow IgG, cow IgM, chicken IgY, and the like, and both the polyclonal and monoclonal antibodies can be used. The antibody fragment is a molecule which has at least one antigen-binding site and derived from a complete antibody, and specific examples thereof include Fab, F(ab')2, and the like. These antibody fragments are obtained by enzymatic treatment or chemical treatment or gene engineering technique.

The method of immobilizing the third binding substances such as antibodies or the substances exhibiting binding properties with respect to the first binding substances onto a substrate is disclosed in, for example, Tech Notes Vol. 2-12 and the like provided from Nunc Laboratory, and any of known methods for preparing general ELISA reagents can be used. Moreover, a self-assembled monolayer (SAM) or the like may be disposed on the substrate to modify the substrate surface. When antibodies are used as the third binding substances, as the principle of immobilizing antibodies onto the substrate, any of principles of physical adsorption and chemical bonding established by covalent bonds can be adopted. As a blocking agent for covering the substrate surface not covered with the antibody even after the antibodies are immobilized onto the substrate, known substances, for example, bovine serum albumin (BSA), skim milk, casein, soybean-derived components, fish-derived components, polyethylene glycol, or commercially available blocking agents for immune reaction that contain the above substances or contain substances having the same property as that of the above substances can be used. These blocking agents can be optionally subjected to pretreatment such as partial denaturation by means of heat, an acid, an alkali, and the like.

### (Drying of first particles and second particles)

In the present invention, the first particles having labels and the second particles not having labels are used in a dry state. Moreover, the kit of the present invention contains the first dry particles having labels and the second dry particles not having labels. Further, the test substance assay reagent of the present invention contains the first dry particles having labels and the second dry particle not having labels. In the present invention, the dry particles refer to particles from which moisture has been removed to such a degree that the mass of moisture (moisture content) based on the mass of solid contents of the particles containing moisture-free labeling substances preferably becomes 30% by mass or less, more preferably become 25% by mass or less, and even more preferably becomes 20% by mass or less. In the present invention, means for drying the particles is not particularly limited, and for example, known drying means such as a drying method using a dehumidifying agent, a drying method implemented under reduced pressure, and a freeze drying method can be used. In the present invention, the first and second particles may be separately dried to obtain dry particles, or alternatively, the first and second particles may be mixed with each other at a desired mass ratio in the state of a solution and then dried to obtain dry particles.

### (Step of trapping test substance)

In the present invention, the test substance is trapped in a reaction site on the substrate which has the third binding substances having binding properties specific to the test substance. The test substance may be trapped in the reaction site by means of any of a sandwich method and a competitive method. The method of trapping the test substance by the sandwich method and the competitive method can be implemented by, for example, the method of general ELISA assay (that is, the method of adding a liquid, in which fluorescent particles and a test substance are dispersed or dissolved, to the surface of a substrate such that the test substance is brought into contact with and trapped in the reaction site) disclosed in Experimental Protocols (p. 258) provided from Sigma-Aldrich Co, LLC. Moreover, by placing the site, where the contact occurs, inside a microchannel, a liquid in which particles having labels and the test substance are dispersed or dissolved can flow through the path and come into contact with the reaction site, whereby the test substance can be trapped. Hereinafter, as specific embodiments of the present invention, the sandwich method and the competitive method will be described, but the present invention is not limited to these methods.

### (Sandwich method)

In the present invention, either the sandwich method or the competitive method may be used. However, it is preferable to use the sandwich method for assay. In the sandwich method, for example, the test substance can be measured by the following sequence, though the sequence is not particularly limited. First, first binding substances having binding properties specific to a test substance and third binding substances having binding properties specific to the test substances are prepared in advance. Thereafter, the first binding substances are bonded to first particles having labels so as to prepare "the first particles that are modified with the first binding substances having binding properties specific to the test substance, having an average particle size of 100 nm to 200 nm, and have labels". Subsequently, third binding substances are prepared and immobilized onto a substrate to form a reaction site (test area). In addition, second binding substances not having binding properties specific to the test substance are prepared and bonded to second particles not having labels so as to prepare "the second particles that are modified with the second binding substances not having binding properties specific to the test substance, have an average particle size of 100 nm to 200 nm, and do not have labels". The first particles are mixed with the second particles, and the resultant is put in a container and dried. A test sample (or an extract thereof) likely to contain the test substance is mixed and dissolved together with the dried mixture of the first and second particles, and the liquid obtained by mixing and dissolving as above is applied to a substrate and caused to flow through a channel on the substrate so as to come into contact with the reaction site. When the test sample contains the test substance, in the reaction site, a reaction occurs between the test substance and the first binding substances having bound to the first particles or between the test substance and the third binding substances on the reaction site (when antibodies and antigens are used, an antigen-antibody reaction occurs), and the first particles corresponding to the amount of the test substance are immobilized onto the reaction site. In the sandwich method, after the reaction between the third binding substances immobilized onto the reaction site and the test substance and the reaction between the test substance and the first binding substances having bound to the first particles, the substrate can be washed for the purpose of removing the first particle which have not bound to the reaction site in two areas on the substrate. Thereafter, signal intensity from the first particles having bound to the reaction site is detected, whereby an accurate concentration of the test substance can be measured. Moreover, there is a positive correlation between the fluorescence intensity and the concentration of the test substance.

### (Competitive method)

In the competitive method, for example, the test substance can be measured by the following sequence, though the sequence is not particularly limited. In the related art, the competitive method is well known as a technique of detecting antigens of low molecular-weight compounds that cannot be analyzed by assay by means of the sandwich method. First, first binding substances having binding properties specific to a test substance and second binding substances not having binding properties specific to the test substance are prepared in advance. Thereafter, the first binding substances are bonded to first particles, and the second binding substances are bonded to second particles. Moreover, the test substance that exhibits binding properties with respect to the first binding substances, or a compound that has a site similar to the test substance and has an epitope which is recognized by the first binding substances similarly to the test substance is immobilized onto a substrate to form a reaction site. Subsequently, the first particles are mixed with the second particles, and the resultant is put in a container and dried. A test sample (or an extract thereof) likely to contain the test substance is mixed and dissolved together with the dried mixture of the first and second particles, and the liquid obtained by mixing and dissolving as above is applied to a substrate and caused to flow through a channel on the substrate so as to come into contact with the reaction site. If the test sample does not contain the test substance, on the substrate, the first binding substances having bound to the first particles react with the test substance, which has been immobilized onto the reaction site and has binding properties specific to the first binding substances, or with the compound which has an epitope that is recognized by the antibodies as the first binding substances similarly to the test substance. On the contrary, if the test sample contains the test substance, the test substance binds to the first binding substances. Accordingly, the reaction caused between the first binding substances and the test substance, which exhibits binding properties with respect to the first binding substances, or the compound, which has a site similar to the test substance and has an epitope that is recognized by the antibodies as the first binding substances similarly to the test substance, on the reaction site is hindered, whereby immobilization of the first particles having labels onto the reaction site is hindered. In the competitive method, plural samples which have different test substance concentrations and of which the test substance mass is known are prepared in advance. While the sample and the fluorescent particles labeling the binding substances are being brought into contact with the reaction site, the fluorescence signals from the reaction site are measured at plural different times. From the measured plural results, temporal change (slope) in the amount of fluorescence at each test substance concentration is determined. A graph in which the Y-axis presents the temporal change and the X-axis presents the test substance concentration is plotted, and an appropriate fitting method such as a method of least squares is used, whereby a calibration curve of the test substance concentration relative to the temporal change of the amount of fluorescence is obtained. Based on the calibration curve obtained in this manner, it is possible to determine the amount of the test substance contained in a test sample from the result of the temporal change of the amount of fluorescence using a target test sample.

### (Channel)

In a preferable embodiment of the present invention, the mixed solution obtained by mixing the test sample (or an extract thereof) which is likely to contain the test substance with the first dry particles having labels and the second dry particles can be applied onto the substrate and caused to flow through a channel. The channel is not particularly limited as long as it acts as a path that enables the test sample, the first particles having labels, and the second particles to flow down to the reaction site. Preferable embodiments of the channel include a structure which has a drip-dispense port into which the test sample solution containing the first particles having labels and the second particles is dispensed and a thin metal film as a reaction site to which the third binding substances have been immobilized, in which a channel extends beyond the thin metal film, and a test sample can pass through the top of the thin metal film. Preferably, it is possible to place an aspiration port in the thin metal film, at the side opposite to the drip-dispense port.

### (Substrate)

As the substrate used for conducting a fluorimetric detection method implemented by surface plasmon excitation (SPF method) which will be described later, it is preferable to use a substrate having the aforementioned channel and a thin metal film as a reaction site on the surface thereof. As the metal composing the thin metal film, substances that can cause surface plasmon resonance can be used. Preferable examples of thereof include free-electron metals such as gold, silver, copper, aluminum, and platinum, and among these, gold is particularly preferable. These metals can be used alone or used in combination. Moreover, in consideration of the adhesiveness of the film to the substrate, an intermediate layer made of chromium or the like may be disposed between the substrate and the layer made of the metal. The film thickness of the metal film is not particularly limited, but it is preferably from 0.1 nm to 500 nm, more preferably from 1 nm to 200 nm, and particularly preferably from 1 nm to 100 nm. If the film thickness exceeds 500 nm, the phenomenon of surface plasmon caused in a medium cannot be detected to a sufficient extent. When the intermediate layer made of chromium or the like is disposed, the thickness of the intermediate layer is preferably from 0.1 nm to 10 nm.

The thin metal film may be formed by conventional methods, for example, a sputtering method, a vapor deposition method, an ion plating method, an electroplating method, and an electroless plating method. In the present invention, it is preferable to form the thin metal film by a sputtering method.

It is preferable for the thin metal film to be disposed on the substrate. Herein, "disposed on the substrate" includes not only the case where the thin metal film is disposed so as to come into direct contact with the substrate but also the case the thin metal film is disposed on the substrate via another layer without coming into direct contact with the substrate. As the substrate usable in the present invention, for example, it is possible to use substrates made of optical glass such as BK7 (borosilicate glass), which is a sort of general optical glass, or other synthetic resins, specifically, polymethyl methacrylate, polyethylene terephthalate, polycarbonate, cycloolefin polymers, and the like that are materials transparent to laser beam. It is desirable for these substrates to be made of materials that do not exhibit anisotropy to polarized light and are excellent in processability. An example of the substrate for fluorescence detection by the SPF method includes a substrate obtained by preparing a gold film on polymethyl methacrylate (PMMA) by a sputtering method and the like.

### (Fluorimetric detection method)

As the fluorimetric detection method of the present invention, it is preferable to detect fluorescence intensity using, for example, an instrument that can detect fluorescence intensity, specifically, a microplate reader or a biosensor for performing the fluorimetric detection method implemented by surface plasmon excitation (SPF method). The detection of fluorescence intensity is usually completed within a certain time after an antigen-antibody reaction, for example, within several minutes to several times after the reaction. To what extent the immunocomplex has been formed is detected in the form of fluorescence intensity, and this makes it possible to determine the concentration of the test substance from the relationship between the fluorescence intensity and the concentration of the test substance. Moreover, the fluorescence may be measured using a plate reader or by means of flow cytometry. Furthermore, with the SPF method, a test substance can be measured with high sensitivity compared to the fluorimetric detection method implemented by epi-illumination excitation (hereinafter, referred to as an "epi-illumination fluorimetry").

As the aforementioned biosensor for surface plasmon fluorimetry (SPF), for example, it is possible to use the sensor which is disclosed in JP2008-249361A and includes an optical waveguide that is formed of a material transmitting excitation light of a predetermined wavelength, a thin metal film that is formed on one surface of the optical waveguide, an light source that generates a light beam, an optical system that causes the light beam to pass through the optical waveguide and to enter the interface between the optical waveguide and the thin metal film at an incidence angle for generating surface plasmon, and fluorescence detection means that detects fluorescence generated by being excited with an evanescent wave enhanced by the surface plasmon.

### (Method of assaying amount of test substance)

In the SPF method of the present invention, the amount of the test substance can be determined by, for example, the following method. Specifically, samples containing test substances of which the concentration is known are prepared, and while the site from which fluorescence is to be detected is caused to flow down, fluorescence signals from the fluorescence detection site are measured at plural different times. From the measured plural results, temporal change (slope) in the amount of fluorescence at each test substance concentration is determined. A graph in which the Y-axis presents the temporal change and the X-axis presents the test substance concentration is plotted, and an appropriate fitting method such as a method of least squares is used, whereby a calibration curve of the test substance concentration relative to the temporal change of the amount of fluorescence is obtained. Regarding the optical signal system, based on the calibration curve corresponding to each of the test substances, the mass of the test substance of the target test sample can be identified.

The system of fluorimetric detection implemented by surface plasmon excitation (SPF) of the present invention is an assay that detects fluorescence from the fluorescent substance that depends on the amount of the test substance immobilized onto the thin metal film on a substrate. This is a method different from the so-called latex agglutination method in which the change in optical transparency that is caused as the reaction in a solution progresses is detected in the form of, for example, turbidity. In the latex agglutination method, antibody-sensitized latex in a latex reagent binds to antigens in a specimen by an antigen-antibody reaction, whereby agglutination occurs. The lump formed by the agglutination grows bigger over time. In the latex agglutination, the lump formed by the agglutination is irradiated with near-infrared light, and from the thus obtained change in absorbance per unit time, antigen concentration is quantified. According to the present invention, it is possible to provide an extremely convenient test substance detection method compared to the latex agglutination method.

### (Kit)

According to the present invention, there is provided a kit for performing a test substance assay including:
first dry particles that are modified with first binding substances having binding properties specific to a test substance, have an average particle size of 100 nm to 200 nm, and have labels;
second dry particles that are modified with second binding substances not having binding properties specific to the test substance, have an average particle size of 100 nm to 200nm, and do not have labels;
a container containing the first dry particles and the second dry particles;
a channel through which the first dry particles and the second dry particles flow; and
a substrate that has third binding substances having binding properties specific to the test substance or has substances exhibiting binding properties with respect to the first binding substances. Preferable embodiments of the kit for test substance assay are as described above in the present specification. The test substance assay method according to the present invention can be implemented using the aforementioned kit for test substance assay.

### (Test substance assay reagent)

Moreover, according to the present invention, there is provided a test substance assay reagent including (a) first dry particles that are modified with first binding substances having binding properties specific to a test substance, have an average particle size of 100 nm to 200 nm, and have labels; and (b) second dry particles that are modified with second binding substances not having binding properties specific to the test substance, have an average particle size of 100 nm to 200 nm, and do not have labels. The test substance assay method according to the present invention can be implemented using the aforementioned test substance assay reagent.

The present invention will be described in more detail by the following examples, but the present invention is not limited to the examples.

### Examples

### 1. Production of latex particles having average particle size of 260 nm

30 g (288 mmol) of styrene (manufactured by Wako Pure Chemical Industries, Ltd.) and 1 g (12 mmol) of acrylic acid (manufactured by Wako Pure Chemical Industries, Ltd.) were suspended in 330 mL of ultrapure water, the resultant was heated to 85°C, and an aqueous solution obtained by dissolving 1 g of potassium persulfate (KPS) (manufactured by Wako Pure Chemical Industries, Ltd.) in 25 mL of water was added thereto, followed by stirring at 85°C for 6 hours at 250 rpm. Thereafter, centrifugation was performed three times on the resultant at 10,000 rpm for 6 hours, thereby obtaining latex particles. Finally, the obtained latex particles were dispersed again in ultrapure water. Pure water was added thereto to prepare a diluted solution having a solid content concentration of 1% by mass. , The average particle size of the latex particles (a median diameter (d = 50) at 25°C using a particle size analyzer FPAR-1000 (OTSUKA ELECTRONICS CO., LTD.)) was confirmed to be 260 nm.

### 2. Production of latex particles having average particle size of 150 nm

30 g (288 mmol) of styrene (manufactured by Wako Pure Chemical Industries, Ltd.) and 3 g (42 mmol) of acrylic acid (manufactured by Wako Pure Chemical Industries, Ltd.) were suspended in 440 mL of ultrapure water, the resultant was heated to 95°C, and an aqueous solution obtained by dissolving 1 g of potassium persulfate (KPS) (manufactured by Wako Pure Chemical Industries, Ltd.) in 10 mL of water was added thereto, followed by stirring at 95°C for 6 hours at 250 rpm. Thereafter, centrifugation was performed three times on the resultant at 10,000 rpm for 6 hours, thereby obtaining latex particles. Finally, the obtained latex particles were dispersed again in ultrapure water. Pure water was added thereto to prepare a diluted solution having a solid content concentration of 1% by mass. , The average particle size of the latex particles (a median diameter (d = 50) at 25°C using a particle size analyzer FPAR-1000 (OTSUKA ELECTRONICS CO., LTD.)) was confirmed to be 150 nm.

Moreover, latex particles having average particle sizes of 140 nm and 170 nm were prepared in the same manner as in Production of latex particles having average particle size of 150 nm, except that the temperature at the time of heating was appropriately adjusted in Production of latex particles having average particle size of 150 nm. The average particle size was measured in the same manner as in the above section 1.

### 3. Preparation of fluorescent latex particles

100 mL of methanol was added to 100 mL of the aqueous dispersion of latex particles prepared as above that had a solid content concentration of 2 % by mass, and the resultant was stirred for 10 minutes at room temperature. Meanwhile, a fluorescence dye (NK136, manufactured by Hayashibara Biochemistry Laboratory) solution (obtained by dissolving the dye in 1 mL of DMF, 9 mL of CHCl₃, and 16mL of EtOH) that was separately prepared was slowly added dropwise to the latex solution over 60 minutes. After the dropwise addition ended, the organic solvent was evaporated under reduced pressure using an evaporator, and the resultant was purified by being subjected three times to centrifugation and resdispersion in an aqueous PBS solution, thereby preparing fluorescent latex particles.

### 4. Preparation of fluorescent latex particles modified with anti-TSH antibodies

Fluorescent particles modified with anti-TSH antibodies were prepared as below.

250 µL of a 50 mM MES buffer (pH 6.0) solution was added to 250 µL of a 2% by mass (solid content concentration) aqueous fluorescent latex particle solution (average particle size of 150 nm), and 100 µL of 5 mg/mL anti-TSH monoclonal antibodies (manufactured by Meridian Life Science, Inc.; Anti-TSH MAb MAT04-410) were added thereto, followed by stirring at room temperature for 15 minutes. Thereafter, 5 µL of a 10 mg/mL aqueous EDC (1-ethyl-3-(3-dimethylaminopropyl)carbodiimide hydrochloride, manufactured by Wako Pure Chemical Industries, Ltd.) solution was added to the resultant, followed by stirring at room temperature for 2 hours. 25 µL of a 2 mol/L aqueous Glycine (manufactured by Wako Pure Chemical Industries, Ltd.) solution was added thereto, and then the resultant was stirred for 30 minutes and subjected to centrifugation (15,000 rpm, 4°C, 15 minutes) to precipitate fluorescent latex particles. Subsequently, the supernatant was removed, 500 µL of a PBS solution (pH 7.4) was added to the resultant, and the fluorescent latex particles were dispersed again by an ultrasonic washing machine. Centrifugation (15,000 rpm, 4°C, 15 minutes) was performed again on the dispersion, and the supernatant was removed. Thereafter, 500 µL of PBS (pH 7.4) solution containing 1% by mass of BSA was added to the resultant, and the fluorescent latex particles were dispersed again, thereby preparing a 1% by mass solution of fluorescent latex particles modified with anti-TSH antibodies. The fluorescent latex particles having an average particle size of 260 nm were also modified with the anti-TSH antibody in the same manner as described above.

### 5. Preparation of particles not labeled with fluorescence

Latex particles modified with anti-T4 antibodies were prepared as below.

250 µL of a 50 mM MES buffer (pH 6.0) solution was added to 250 µL of a 2% by mass (solid content concentration) aqueous latex particle solution (average particle size of 150 nm), and 100 µL of 5 mg/mL anti-T4 monoclonal antibodies (manufactured by Medix Biochemica, Anti-Thyroxine monoclonal antibodies (6901)) were added thereto, followed by stirring at room temperature for 15 minutes. Thereafter, 5 µL of a 10 mg/mL aqueous EDC solution was added to the resultant, followed by stirring at room temperature for 2 hours. 25 µL of a 2 mol/L aqueous Glycine (manufactured by Wako Pure Chemical Industries, Ltd.) solution was added thereto, and then the resultant was stirred for 30 minutes and subjected to centrifugation (15,000 rpm, 4°C, 15 minutes) to precipitate latex particles. Subsequently, the supernatant was removed, 500 µL of a PBS solution (pH 7.4) was added to the resultant, and the latex particles were dispersed again by an ultrasonic washing machine. Centrifugation (15,000 rpm, 4°C, 15 minutes) was performed again on the dispersion, and the supernatant was removed. Thereafter, 500 µL of PBS (pH 7.4) solution containing 1% by mass of BSA was added to the resultant, and the latex particles were dispersed again, thereby preparing a 1% by mass solution of fluorescent latex particles modified with anti-T4 antibodies. The latex particles having an average particle size of 260 nm were also modified with the anti-T4 antibody in the same manner as described above.

Using anti-hCG antibodies (manufactured by Medix Biochemica, Anti-hCG beta monoclonal antibodies (5008)), the latex particles having an average particle size of 150 nm were also modified with the anti hCG antibodies in the same manner as described above.

### 6. Preparation of fluorescence-labeled dry particles and dry particles not labeled with fluorescence

280 µL of ultrapure water, 427 µL of a 12.5% by mass aqueous sucrose solution, 133 µL of a 20% by mass aqueous BSA solution, 80 µL of 1% by mass fluorescent latex particles (average particle size of 150 nm) modified with anti-TSH antibodies, and 80 µL of 1% by mass latex particles (average particle size of 150 nm) modified with anti-T4 antibodies were mixed together. A cup made of polypropylene (manufactured by Prime Polymer Co., Ltd., Prime Polypro random PP grade) as a base material was prepared, and 15 µL of the mixture was dripped into the cup. Thereafter, using a drying machine Super Dry (manufactured by TOYO LIVING CO., LTD, Ultrasuper Dry 00 series), the mixture was dried until over 12 hours until the moisture content thereof became 25% or less, thereby preparing dry particles described in Experiment level 5 shown in Table 1. Regarding dry particles used for other Experiment levels, dry particles of Experiment levels 1 to 11 were prepared by appropriately changing the average particle size of the latex particles and the amount thereof used.

### 7. Preparation of substrate

On one surface of a base material made of polymethyl methacrylate (PMMA, manufactured by MITSUBISHI RAYON CO., LTD., Acrypet VH-001), a gold film for a test area and a gold film for a control area that was adjacent to the above gold film were prepared in a horizontal direction by sputtering (both the gold films had a width of 4 mm and a thickness of 45 nm). The base material including the gold films was cut in a vertical direction (a width of 5 mm) to prepare a substrate. On the gold film of the substrate that was vapor-deposited and used as a test area, a solution (concentration: 10 µg/mL in 150 mM NaCl) containing anti-TSH monoclonal antibodies (manufactured by Medix Biochemica, 5409) was dripped, and the antibodies were incubated for 1 hour at 25°C such that they were immobilized by being physically adsorbed onto the substrate.

### 8. Washing of substrate and blocking

Before being mounted on a channel of a sensor chip, the substrate prepared as above was repeatedly washed three times with 300 µL of a PBS solution (pH 7.4) prepared in advance that contained a washing solution (0.05% by mass Tween 20 (polyoxyethylene (20) sorbitan monolaurate, manufactured by Wako Pure Chemical Industries, Ltd.)). After the washing ended, in order to block the antibody-unadsorbed portion on the vapor-deposited gold film, 300 µL of a PBS solution (pH 7.4) containing 1% by mass casein (manufactured by Thermo Scientific) was added to the substrate, and the substrate was allowed to standstill for 1 hour at room temperature. After the substrate was washed with the aforementioned washing solution, 300 µL of Immunoassay Stabilizer (manufactured by Advanced Biotechnologies Inc) was added thereto as a stabilizer, the substrate was allowed to standstill at room temperature for 30 minutes to remove the solution, and moisture thereof was completely removed using a drying machine.

### 9. Preparation of sensor chip

The prepared substrate was enclosed in a channel so that have the configuration disclosed in the second embodiment of JP2010-190880A, thereby preparing a channel-type sensor chip.

### 10. Preparation of test sample

As dog serum, the serum of oriental beagle purchased from KITAYAMA LABES CO., LTD. was used to prepare Test sample (Specimen) No. 1 to 11.

### 11. Immunoassay for TSH using fluorescent particles

100 µL of the test sample (dog serum) prepared in the section 10 was thoroughly mixed with 44 µmol magnesium chloride, thereby preparing a mixed sample. The dry particles in the cup that were prepared in the section 6 were stored for 15 days in an environment of 25°C and 50% RH. The aforementioned mixed sample was dripped to the dry particles in an immunochemoluminescence analyzer, and the resultant was mixed with each other under stirring for 10 minutes, thereby obtaining a Mixed solution 1. Thereafter, the obtained Mixed solution 1 was dripped onto the channel-type sensor chip which was prepared in the section 9 and in which the substrate was enclosed. After being dripped onto the sensor chip, the Mixed solution 1 was caused to flow down at a rate of 10 µL/min under aspiration performed using a pump. The fluorescence intensity on the gold-deposited substrate surface onto which the TSH antibodies had been fixed was continuously measured for 1.5 minutes. The increase rate of the obtained fluorescence intensity per unit time was taken as the value of fluorescence signal.

In addition, the dispersions shown in the following 1' and 7' (of Table 1) were dripped in an amount of 15 µl into the cup used in the section 6. Then, the cup was sealed and stored for 15 days in an environment of 25°C and 50% RH without being subjected to drying. The aforementioned mixed sample was dripped into the cup in the aforementioned analyzer, and the resultant was mixed under stirring for 10 minutes, thereby obtaining Mixed solutions 2 and 3. The increase rate of the fluorescence intensity per unit time was taken as the value of fluorescence signal in the same manner as described above, except that Mixed solutions 2 and 3 were used instead of the Mixed solution 1.

### 12. Assay performed by control analyzer

Using IMMULYZE 1000 from Siemens AG, which is a large size full automatic immunochemolumenescence analyzer widely used by those in the related art for immunoassay, the test substance in the test sample was measured according to the instruction manual of the analyzer. The present invention is based on the values measured by the control analyzer and makes it possible to more rapidly and conveniently measure the test substance. The present invention aims to reduce the difference in the assay value between the present invention and the control analyzer. The difference in the assay value between the present invention and the control analyzer was evaluated according to the following criteria and described in Table 1.

Criteria for evaluating improvement of non-specificity:
E: Level of test substance dissociated from the control analyzer (IMMULYZE from Siemens AG) is 15 ng/mL or higher.
D: Level of test substance dissociated from the control analyzer (IMMULYZE from Siemens AG) is 10 ng/mL or higher and less than 15 ng/mL.
C: Level of test substance dissociated from the control analyzer (IMMULYZE from Siemens AG) is 1 ng/mL or higher and less than 10 ng/mL.
B: Level of test substance dissociated from the control analyzer (IMMULYZE from Siemens AG) is 0.6 ng/mL or higher and less than 1 ng/mL.
A: Level of test substance dissociated from the control analyzer (IMMULYZE from Siemens AG) is less than 0.6 ng/mL.

### 13. Assay of degree of signal variation (CV)

Regarding CV of signals, a standard deviation (SD value) found when a signal value of the test area was obtained under the condition of N = 10 was divided by the average value, and the result was multiplied by 100 and indicated as CV. As the evaluation criteria, a case where CV ≤ 10% was evaluated to be a, and a case where CV > 10% was evaluated to be b. The evaluation results are described in the table.

The results obtained using Specimen 1, from which false positives were detected, are described in Table 1. The assay result obtained by IMMULYZE of Siemens AG was 0.1 ng/mL.

**[Table 1]**

| Experime nt level | State of reagent | Specimen No. | Average particle size of labeled particles /nm | Average particle size of unlabeled particles/n m | Ratio of labeled particles/unlabele d particles | Type of mouse antibody of unlabeled particles | Improvement of non-specificity ng/mL | Signal CV | Note |
|---|---|---|---|---|---|---|---|---|---|
| 1 | Dry | 1 | 260 | - | 1/0 | - | E 15.3 | b 25% | Comparative example |
| 1' | Dispersi on | 1 | 260 | - | 1/0 | - | E 15.1 | b 28% | Comparative example |
| 2 | Dry | 1 | 150 | - | 1/0 | - | D 12.1 | a 8% | Comparative example |
| 3 | Dry | 1 | 260 | 260 | 1/1 | 1 | C 8.3 | b 20% | Comparative example |
| 4 | Dry | 1 | 260 | 150 | 1/1 | 1 | B 0.75 | b 24% | Comparative example |
| 5 | Dry | 1 | 150 | 150 | 1/1 | 1 | A 0.57 | a 8% | Comparative invention |
| 6 | Dry | 1 | 150 | 150 | 1/2 | 1 | A 0.22 | a 7% | Present invention |
| 7 | Dry | 1 | 150 | 150 | 1/4 | 1 | A 0.03 | a 4% | Present invention |
| 7' | Dispersi on | 1 | 150 | 150 | 1/4 | 1 | A 0.11 | b 16% | Comparative example |
| 8 | Dry | 1 | 150 | 150 | 1/6 | 1 | A 0.07 | a 6% | Present invention |
| 9 | Dry | 1 | 140 | 140 | 1/4 | 1 | A 0.04 | a 8% | Present invention |
| 10 | Dry | 1 | 170 | 170 | 1/4 | 1 | A 0.05 | a 10% | Present invention |
| 11 | Dry | 1 | 150 | 150 | 1/4 | 2 | A 0.03 | a 6% | Present invention |

### (Type of mouse antibody)

1: Anti-Thyroxine monoclonal antibody (6901) from Medix Biochemica
2: Anti-hCG beta monoclonal antibody (5008) from Medix Biochemica

From the results shown in Table 1, it was confirmed that the use of the labeled particles and unlabeled particles having the average particle size specified by the present invention reduces the difference in the assay value between the present invention and the control analyzer. Particularly, in Experiment levels 6 to 11 in which the ratio of the unlabeled particles to the labeled particles is 1/2 to 1/6, the TSH level measured by immunoassay excellently matched with the level measured by the control analyzer. Moreover, it was confirmed that the use of the fluorescence-labeled dry particles having an average particle size within the range specified by the present invention reduces the variation (signal CV) of the assay, compared to the case where labeled particles in a solution state or labeled particles having an average particle size of 200 nm or greater are used.

### Example 2

The dry particles 7 used in Example 1 and dry particles which were obtained by mixing 260 nm of labeled particles with 260 nm of unlabeled particles at a ratio of 1/4 were prepared in the method used in the above section 6. Using these particles and the test samples of Specimen Nos. 2 to 7, experiments for confirming the effects of the present invention were performed. The experiment and assay were performed in the same manner as in Example 1.

**[Table 2]**

| | | | | | | |
|---|---|---|---|---|---|---|
| Results obtained using dry particles 7 | | | | | | |

| Specimen No. | Average particle size of labeled particles/nm | Average particle size of unlabeled particles/nm | Ratio of labeled particles/unlabeled particles | Improvement of Signal non-specificity CV ng/mL | | Note |
|---|---|---|---|---|---|---|
| 2 | 150 | 150 | 1/4 | A 0.3 | a 4% | Present invention |
| 3 | 150 | 150 | 1/4 | A 0.0 | a 6% | Present invention |
| 4 | 150 | 150 | 1/4 | A 0.0 | a 7% | Present invention |
| 5 | 150 | 150 | 1/4 | A 0.1 | a 6% | Present invention |
| 6 | 150 | 150 | 1/4 | A 0.1 | a 9% | Present invention |
| 7 | 150 | 150 | 1/4 | A 0.0 | a 8% | Present invention |

**[Table 3]**

| | | | | | | |
|---|---|---|---|---|---|---|
| Results obtained using particles having average particle size of 260 nm | | | | | | |

| Specimen No. | Average particle size of labeled particles/nm | Average particle size of unlabeled particles/mn | Ratio of labeled particles/unlabeled particles | Improvement of non-specificity ng/mL | Signal CV | Note |
|---|---|---|---|---|---|---|
| 2 | 260 | 260 | 1/4 | C 1.7 | b 16% | Comparative example |
| 3 | 260 | 260 | 1/4 | C 1.5 | b 30% | Comparative example |
| 4 | 260 | 260 | 1/4 | C 1.1 | b 17% | Comparative example |
| 5 | 260 | 260 | 1/4 | B 0.7 | b 22% | Comparative example |
| 6 | 260 | 260 | 1/4 | B 0.6 | b 27% | Comparative example |
| 7 | 260 | 260 | 1/4 | C 1.0 | b 15% | Comparative example |

It was confirmed that using unlabeled particles having an average particle size of 150 nm for plural specimens, the difference in the assay value between the present invention and the control analyzer is reduced. Moreover, it was confirmed that the use of the fluorescence-labeled particles having an average particle size of 150 nm reduces the variation (signal CV) of assay.

## Claims

1. A test substance assay method comprising:
(i) forming a mixed solution by mixing (a) first dry particles that are fluorescent latex particles with an average particle size of 100 nm to 200 nm and modified with first binding substances which are antibodies, antigens or complexes of these having binding properties specific to a test substance, and (b) second dry particles that are non-fluorescent latex particles with an average particle size of 100 nm to 200 nm and modified with second binding substances which are antibodies not having binding properties specific to the test substance, with (c) a test sample solution containing the test substance, the mass ratio of the second dry particles to the first dry particles being 2:1 to 6:1;
(ii) applying the mixed solution obtained in the step (i) onto a substrate having a reaction site, the reaction site having third binding substances which are antibodies, antigens or complexes of these with binding properties specific to the test substance; or has substances exhibiting binding properties with respect to the first binding substances;
(iii) causing the test substance to be trapped in the reaction site on the substrate; and
(iv) detecting the test substance trapped in the reaction site.

2. A method according to Claim 1, wherein the first binding substances having binding properties specific to the test substance are antibodies.

3. A method according to Claim 1 or Claim 2, wherein the first binding substances having binding properties specific to the test substance are antibodies derived from a mouse.

4. A method according to any preceding claim, wherein, in the step (iv), the trapping of the test substance in the reaction site is detected by surface plasmon fluorimetry.

5. A kit for performing a test substance assay comprising:
first dry particles that are fluorescent latex particles with an average particle size of 100 nm to 200 nm and modified with first binding substances which are antibodies, antigens or complexes of these having binding properties specific to a test substance;
second dry particles that are non-fluorescent latex particles with an average particle size of 100 nm to 200 nm and modified with second binding substances which are antibodies not having binding properties specific to the test substance, the mass ratio of the second dry particles to the first dry particles being 2:1 to 6:1;
a container containing the first dry particles and the second dry particles;
a channel through which the first dry particles and the second dry particles are capable of flowing; and
a substrate that has third binding substances which are antibodies, antigens or complexes of these having binding properties specific to the test substance; or has substances exhibiting binding properties with respect to the first binding substances.

6. A kit according to Claim 5, wherein the first binding substances having binding properties specific to the test substance are antibodies.

7. A kit according to Claim 5 or Claim 6, wherein the first binding substances having binding properties specific to the test substance are antibodies derived from a mouse.

8. A method according to any of Claims 1 to 4 that is implemented using the kit according to any of Claims 5 to 7.

9. A test substance assay reagent comprising:
(a) first dry particles that are fluorescent latex particles with an average particle size of 100 nm to 200 nm and modified with first binding substances which are antibodies, antigens or complexes of these having binding properties specific to a test substance; and
(b) second dry particles that are non-fluorescent latex particles with an average particle size of 100 nm to 200 nm and modified with second binding substances which are antibodies not having binding properties specific to the test substance, the mass ratio of the second dry particles to the first dry particles being 2:1 to 6:1.

10. A test substance assay reagent according to Claim 9, wherein the first binding substances having binding properties specific to the test substance are antibodies.

11. A test substance assay reagent according to Claim 9 or Claim 10, wherein the first binding substances having binding properties specific to the test substance are antibodies derived from a mouse.

## Patentansprüche

1. Testsubstanz- Assay-Verfahren, umfassend:
(i) Bilden einer gemischten Lösung durch Mischen von (a) ersten trockenen Partikeln, die fluoreszierende Latexpartikel mit einer mittleren Partikelgröße von 100 nm bis 200 nm sind und mit ersten Bindungssubstanzen modifiziert sind, die Antikörper, Antigene oder Komplexe davon mit für eine Testsubstanz spezifischen Bindungseigenschaften sind, und (b) zweiten trockenen Partikeln, bei denen es sich um nicht-fluoreszierende Latexpartikel mit einer mittleren Partikelgröße von 100 nm bis 200 nm handelt und die mit zweiten Bindungssubstanzen modifiziert sind, bei denen es sich um Antikörper handelt, die keine für die Testsubstanz spezifischen Bindungseigenschaften aufweisen, mit (c) einer Testprobenlösung, die die Testsubstanz enthält, wobei das Massenverhältnis der zweiten trockenen Partikel zu den ersten trockenen Partikeln 2:1 bis 6:1 beträgt;
(ii) Aufbringen der in Schritt (i) erhaltenen Mischlösung auf ein Substrat mit einer Reaktionsstelle, wobei die Reaktionsstelle dritte Bindungssubstanzen aufweist, die Antikörper, Antigene oder Komplexe davon mit für die Testsubstanz spezifischen Bindungseigenschaften sind; oder die Substanzen aufweist, die Bindungseigenschaften in Bezug auf die ersten Bindungssubstanzen aufweisen;
iii) Bewirken, dass die Testsubstanz in der Reaktionsstelle auf dem Substrat eingeschlossen wird; und
(iv) Detektieren der in der Reaktionsstelle eingeschlossenen Testsubstanz.

2. Verfahren gemäß Anspruch 1, wobei die ersten Bindungssubstanzen mit für die Testsubstanz spezifischen Bindungseigenschaften Antikörper sind.

3. Verfahren gemäß Anspruch 1 oder Anspruch 2, wobei die ersten Bindungssubstanzen mit für die Testsubstanz spezifischen Bindungseigenschaften Antikörper sind, die von einer Maus stammen.

4. Verfahren gemäß einem der vorstehenden Ansprüche, wobei in Schritt (iv) das Einschließen der Testsubstanz in die Reaktionsstelle durch Oberflächenplasmonenfluorimetrie detektiert wird.

5. Kit zur Durchführung eines Testsubstanz-Assays, umfassend:
erste trockene Partikel, die fluoreszierende Latexpartikel mit einer mittleren Partikelgröße von 100 nm bis 200 nm sind und mit ersten Bindungssubstanzen modifiziert sind, die Antikörper, Antigene oder Komplexe davon mit für eine Testsubstanz spezifischen Bindungseigenschaften sind;
zweite trockene Partikel, die nicht-fluoreszierende Latexpartikel mit einer mittleren Partikelgröße von 100 nm bis 200 nm sind und mit zweiten Bindungssubstanzen modifiziert sind, die Antikörper sind, die keine für die Testsubstanz spezifischen Bindungseigenschaften aufweisen, wobei das Massenverhältnis der zweiten trockenen Partikel zu den ersten trockenen Partikeln 2:1 bis 6:1 beträgt;
einen Behälter, der die ersten trockenen Partikel und die zweiten trockenen Partikel enthält;
einen Kanal, durch den die ersten trockenen Partikel und die zweiten trockenen Partikel fließen können; und
ein Substrat, das dritte Bindungssubstanzen aufweist, bei denen es sich um Antikörper, Antigene oder Komplexe davon mit für die Testsubstanz spezifischen Bindungseigenschaften handelt; oder das Substanzen aufweist, die Bindungseigenschaften in Bezug auf die ersten Bindungssubstanzen aufweisen.

6. Kit gemäß Anspruch 5, wobei die ersten Bindungssubstanzen mit für die Testsubstanz spezifischen Bindungseigenschaften Antikörper sind.

7. Kit gemäß Anspruch 5 oder Anspruch 6, wobei die ersten Bindungssubstanzen mit für die Testsubstanz spezifischen Bindungseigenschaften Antikörper sind, die von einer Maus stammen.

8. Verfahren gemäß einem der Ansprüche 1 bis 4, das unter Verwendung des Kits gemäß einem der Ansprüche 5 bis 7 durchgeführt wird.

9. Testsubstanz-Assay-Reagenz, umfassend:
(a) erste trockene Partikel, die fluoreszierende Latexpartikel mit einer durchschnittlichen Partikelgröße von 100 nm bis 200 nm sind und mit ersten Bindungssubstanzen modifiziert sind, die Antikörper, Antigene oder Komplexe davon mit für eine Testsubstanz spezifischen Bindungseigenschaften sind; und
(b) zweite trockene Partikel, bei denen es sich um nicht-fluoreszierende Latexpartikel mit einer durchschnittlichen Partikelgröße von 100 nm bis 200 nm handelt und die mit zweiten Bindungssubstanzen modifiziert sind, bei denen es sich um Antikörper handelt, die keine für die Testsubstanz spezifischen Bindungseigenschaften aufweisen,
wobei das Massenverhältnis der zweiten trockenen Partikel zu den ersten trockenen Partikeln 2:1 bis 6:1 beträgt.

10. Testsubstanz-Assay-Reagenz gemäß Anspruch 9, wobei die ersten Bindungssubstanzen mit für die Testsubstanz spezifischen Bindungseigenschaften Antikörper sind.

11. Reagenz zum Testen von Testsubstanzen gemäß Anspruch 9 oder Anspruch 10, wobei die ersten Bindungssubstanzen mit für die Testsubstanz spezifischen Bindungseigenschaften Antikörper sind, die von einer Maus stammen.

## Revendications

1. Procédé de dosage de substance d'essai, comprenant les étapes consistant à :
(i) former une solution mélangée en mélangeant (a) des premières particules sèches qui sont des particules en latex fluorescentes avec une taille de particule moyenne de 100 nm à 200 nm et modifiées avec des premières substances de liaison qui sont des anticorps, des antigènes ou des complexes de ceux-ci présentant des propriétés de liaison spécifiques à une substance d'essai, et (b) des secondes particules sèches qui sont des particules en latex non fluorescentes avec une taille de particule moyenne de 100 nm à 200 nm et modifiées avec des deuxièmes substances de liaison qui sont des anticorps ne présentant pas de propriétés de liaison spécifiques à la substance d'essai, avec (c) une solution d'échantillon d'essai contenant la substance d'essai, le rapport de masse entre les secondes particules sèches et les premières particules sèches étant de 2:1 à 6:1 ;
(ii) appliquer la solution mélangée obtenue à l'étape (i) sur un substrat présentant un site de réaction, le site de réaction présentant des troisièmes substances de réaction qui sont des anticorps, des antigènes ou des complexes de ceux-ci avec des propriétés de liaison spécifiques à la substance d'essai ; ou présente des substances manifestant des propriétés de liaison par rapport aux premières substances de liaison ;
(iii) amener la substance d'essai à être retenue dans le site de réaction sur le substrat ; et
(iv) détecter la substance d'essai retenue dans le site de réaction.

2. Procédé selon la revendication 1, dans lequel les premières substances de liaison présentant des propriétés de liaison spécifiques à la substance d'essai sont des anticorps.

3. Procédé selon la revendication 1 ou la revendication 2, dans lequel les premières substances de liaison présentant des propriétés de liaison spécifiques à la substance d'essai sont des anticorps dérivés d'une souris.

4. Procédé selon une quelconque revendication précédente, dans lequel, dans l'étape (iv), la retenue de la substance d'essai dans le site de réaction est détectée par une fluorimétrie plasmonique de surface.

5. Kit pour effectuer un dosage de substance d'essai comprenant :
des premières particules sèches qui sont des particules en latex fluorescentes avec une taille de particule moyenne de 100 nm à 200 nm et modifiées avec des premières substances de liaison qui sont des anticorps, des antigènes ou des complexes de ceux-ci présentant des propriétés de liaison spécifiques à une substance d'essai ;
des secondes particules sèches qui sont des particules en latex non fluorescentes avec une taille de particule moyenne de 100 nm à 200 nm et modifiées avec des deuxièmes substances de liaison qui sont des anticorps ne présentant pas de propriétés de liaison spécifiques à la substance d'essai, le rapport de masse entre les secondes particules sèches et les premières particules sèches étant de 2:1 à 6:1 ;
un récipient contenant les premières particules sèches et les secondes particules sèches ;
un canal à travers lequel les premières particules sèches et les secondes particules sèches sont capables de circuler ; et
un substrat qui présente des troisièmes substances de liaison qui sont des anticorps, des antigènes ou des complexes de ceux-ci présentant des propriétés de liaison spécifiques à la substance d'essai ; ou présente des substances manifestant des propriétés de liaison par rapport aux premières substances de liaison.

6. Kit selon la revendication 5, dans lequel les premières substances de liaison présentant des propriétés de liaison spécifiques à la substance d'essai sont des anticorps.

7. Kit selon la revendication 5 ou la revendication 6, dans lequel les premières substances de liaison présentant des propriétés de liaison spécifiques à la substance d'essai sont des anticorps dérivés d'une souris.

8. Procédé selon l'une quelconque des revendications 1 à 4 qui est mis en œuvre en utilisant le kit selon l'une quelconque des revendications 5 à 7.

9. Réactif de dosage de substance d'essai comprenant :
(a) des premières particules sèches qui sont des particules en latex fluorescentes avec une taille de particule moyenne de 100 nm à 200 nm et modifiées avec des premières substances de liaison qui sont des anticorps, des antigènes ou des complexes de ceux-ci présentant des propriétés de liaison spécifiques à une substance d'essai ; et
(b) des secondes particules sèches qui sont des particules en latex non fluorescentes avec une taille de particule moyenne de 100 nm à 200 nm et modifiées avec des deuxièmes substances de liaison qui sont des anticorps ne présentant pas de propriétés de liaison spécifiques à la substance d'essai, le rapport de masse entre les secondes particules sèches et les premières particules sèches étant de 2:1 à 6:1.

10. Réactif de dosage de substance d'essai selon la revendication 9, dans lequel les premières substances de liaison présentant des propriétés de liaison spécifiques à la substance d'essai sont des anticorps.

11. Réactif de dosage de substance d'essai selon la revendication 9 ou la revendication 10, dans lequel les premières substances de liaison présentant des propriétés de liaison spécifiques à la substance d'essai sont des anticorps dérivés d'une souris.
